Europäisches Patentamt

European Patent Office

Office européen des brevets

(19)

(11) **EP 1 261 570 B1**

(12) **EUROPEAN PATENT SPECIFICATION**

(45) Date of publication and mention
of the grant of the patent:
**26.10.2005 Bulletin 2005/43**

(51) Int Cl.$^7$: **C07C 5/333**

(86) International application number:
**PCT/NO2001/000026**

(21) Application number: **01946838.8**

(22) Date of filing: **25.01.2001**

(87) International publication number:
**WO 2001/055062 (02.08.2001 Gazette 2001/31)**

(54) **METHOD AND REACTOR FOR AUTOTHERMAL DEHYDROGENATION OF HYDROCARBONS**

VERFAHREN UND REAKTOR ZUR AUTOTHERMISCHEN DEHYDROGENIERUNG VON KOHLENWASSERSTOFFEN

PROCEDE ET REACTEUR DE DESHYDROGENATION AUTOTHERMIQUE D'HYDROCARBURES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **25.01.2000 NO 20000374**

(43) Date of publication of application:
**04.12.2002 Bulletin 2002/49**

(73) Proprietor: **Statoil ASA**
**4035 Stavanger (NO)**

(72) Inventors:
• **RYTTER, Erling**
**N-7049 Trondheim (NO)**

• **OLSBYE, Unni**
**N-1187 Oslo (NO)**
• **SORAKER, Pal**
**N-7049 Trondheim (NO)**
• **TORVIK, Rolf**
**N-7560 Vikhamar (NO)**

(74) Representative: **Rees, David Christopher et al**
**Kilburn & Strode**
**20 Red Lion Street**
**London WC1R 4PJ (GB)**

(56) References cited:
**EP-A1- 0 323 115        WO-A1-96/19424**
**US-A- 4 613 715        US-A- 4 739 124**
**US-A- 4 806 624        US-A- 4 914 249**

## Description

The present invention relates to an improved method for the autothermal or near autothermal catalytic dehydrogenation of hydrocarbons in which oxygen is introduced directly into the catalytic bed. The invention also relates to a reactor for carrying out the method.

## Background of invention

Catalytic dehydrogenation of hydrocarbons, globally described by eq. (i), is a well-known and commercially important process.

$$C_nH_{2n+2} \leftrightarrow C_nH_{2n} + H_2 \qquad\qquad (i)$$

The reaction is strongly endothermal. At adiabatic conditions this will result in a lowering of the temperature in the reaction mixture and a consequently lowering of the reaction rate. Therefore, existing catalytic dehydrogenation processes are dependent on external heat supply to uphold the reaction temperature. Besides, dehydrogenation reactions are subject to equilibrium limitations at typical process conditions.

The above-mentioned limitations have led to the development of autothermal dehydrogenation (ADH) processes wherein the dehydrogenation is effected in combination with an oxidation with an oxygen-containing gas of the generated hydrogen to form water. At typical reaction conditions, the exothermic heat generated by the combustion of about half of the formed hydrogen will compensate for the heat loss resulting from the endothermic dehydrogenation reaction. In addition to achieve a desired heat balance, the consumption of hydrogen in the combustion reaction will shift the equilibrium of the desired dehydrogenation reaction in the direction of a higher conversion to dehydrogenated hydrocarbons.

In the applicants own WO96/19424, a reactor for catalytic dehydrogenation of hydrocarbons wherein hydrogen is specifically oxidised. The reactor comprises a plurality of serial connecfed catalytic zones where oxygen-containing gas is introduced between the catalytic zones. It is emphasised in the publication that the oxygen-containing gas and the gas containing the partly dehydrogenated hydrocarbons has to be well mixed before entering the catalyst bed, and that the mixing time should be sufficiently short to avoid oxidation reactions in the gas phase.

US Patent No. 4,914,249 describes a process for autothermal dehydrogenation of a hydrocarbon, comprising two dehydrogenation stages and an intermediate oxidation stage for a selective oxidation of hydrogen to water. In this process, the effluent stream from the first dehydrogenation stage, comprising a mixture of dehydrogenated hydrocarbon, unconverted hydrocarbon, hydrogen and steam, is subjected to a selective oxidation of hydrogen on a separate oxidation catalyst in a separate oxidation zone, to which zone the oxygen-containing gas required for the combustion is fed preferably at a position adjacent to the bed of oxidation catalyst. The effluent from this separate oxidation zone is then subjected to the next dehydrogenation step.

US Patent No. 4,739,124 discloses an autothermal dehydrogenation process where a hydrocarbon represented by ethane is catalytic dehydrogenated in a reactor comprising a least two separate beds of a dehydrogenation catalyst. A hydrocarbon feed stream, e.g. ethane, is passed into the first bed of dehydrogenation catalyst maintained at dehydrogenation conditions, i.e. at temperatures in the range of 538°C to 750°C. The effluent stream from this first catalytic bed is cooled and then mixed with an oxygen-containing gas in a catalyst-free zone, whereupon the obtained mixture is fed to a separate bed of a selective hydrogen oxidation catalyst maintained at oxidation promoting conditions. The effluent stream from said oxidation bed, which has been heated as a result of the hydrogen combustion, is passed to a second bed of dehydrogenation catalyst similar to the first bed of dehydrogenation catalyst. The purpose of cooling the effluent stream from the first bed of dehydrogenation catalyst, by direct or indirect heat exchange, is to increase the need for combustion of hydrogen in the bed of hydrogen oxidation catalyst. Because a larger part of the hydrogen in the gas mixture has now to be consumed to reach the desired dehydrogenation temperature, the equilibrium concentration of dehydrogenated hydrocarbon in the gas mixture is increased, and the higher equilibrium concentration becomes a driving force for achieving an increased conversion in the dehydrogenation reaction.

DE Patent No. 197 34 541 discloses a dehydrogenation process where the oxygen-containing gas is introduced together with the hydrocarbon at the reactor inlet, and then preheated to a temperature in the range 200°C-650°C. The said gas mixture is then passed over a catalyst which is partly active for oxidation, and partly active for the oxidative dehydrogenation of the hydrocarbon, represented by ethane or propane. The catalyst composition is: $MA_aB_bP_yO_x$, where: M = Sc, Y, La, Nd, Pm, Sm, Eu, Gd, Dy, Ho. Er or Yb; A = Mg, Ca or Sr; A = 0.01-10; B = Li, Na, K or Cs; b = 0-0.2; P = Phosphorus; y = 0-0.1; O = Oxygen; and x is the stoichiometric amount of oxygen. A major point in the choice of a catalyst is to avoid expensive noble metals that are often used in non-oxidative dehydrogenation

**EP 1 261 570 B1**

processes. The primary role of the catalyst is to increase the reactor temperature by combustion of the hydrocarbon. By such oxidation, the reactor temperature increases to 650°C-900°C. After the oxidation reaction has been completed, the hydrocarbon is subject to thermal dehydrogenation, either in an empty reactor, or in the presence of inert particles such as quartz or alpha-alumina.

The oxidation catalyst may be present as particles in a fixed bed state, or as a monolith, whereas the inert particles may be present in a fixed bed state or in a fluidised bed state. Although no such catalyst is mentioned, this patent further proposes that the hydrocarbon dehydrogenation following the oxidation zone could also be performed over a catalyst. The maximum propene yield, which is quoted in the Examples, is 17% (on Mole Carbon basis). This yield is obtained at 750°C and 1 atm.

The prior art in the field of autothermal dehydrogenation reactions (ADH), represented by first three of the patents quoted above have focused on the selective oxidation of hydrogen. According to DE 197 34 541 the necessary temperature is achieved by combustion of hydrocarbons before the dehydrogenation.

Common to WO96/19424, US 4.914.249 and US 4.739.124 are that they comprises separate mixing zones for the mixing of an oxygen containing and a hydrocarbon containing gas stream outside the dehydrogenation catalytic bed and in some instances even outside the reactor. This gives a relatively complex and expensive reactor construction. The exothermal oxidation will then take place in a narrow band of the reactor close to the inlet end of the catalytic bed. The subsequent endothermal dehydrogenation reaction will then result in a temperature gradient of decreasing temperature from the inlet end to the outlet end of the catalytic bed. If the reaction temperature at the inlet side of the catalytic bed is optimal for the reaction, the temperature at the outlet side will as a result be too low for an optimal reaction rate.

US 4.613.715 describes oxygen addition to a steam active dehydrogenation reactor. A first stream of oxygen is added to the hydrocarbon feed stream before entering the catalytic bed and a second stream injected into the catalyst bed.

EP-A-323115 relates to a process for steam dehydrogenation of dehydrogenatable hydrocarbons with simultaneous oxidative reheating. According to the description oxygen containing gas may be added into the catalytic bed.

Even ifboth US 4.613.715 and EP-A-323115 describes the possibility of injection of an oxygen containing gas into the catalytic bed no description on how such an injection is achieved.

**Summary of invention**

One objective of the present invention is therefor to provide an improved method for autothermal catalytic dehydrogenation (ADH) of hydrocarbons where the above-mentioned disadvantages are avoided or minimised.

This object is met by a method for autothermal or substantially autothermal catalytic dehydrogenation of hydrocarbons, where a hydrocarbon containing feed gas optionally is mixed with steam and optionally mixed with hydrogen, is preheated and led into a reactor comprising a dehydrogenation catalytic bed and where a oxygen containing gas is fed into the hydrocarbon containing feed gas directly in the catalytic bed, wherein the oxygen containing gas is fed into the catalytic bed from one or more oxygen supply tube(s) having a plurality of openings distributed in the catalytic bed.

Preferably, the openings in a oxygen supply tube are situated around the circumference and in the longitudinal direction of the oxygen supply tube.

According to a preferred embodiment the openings in the oxygen supply tube is a membrane having pores allowing oxygen to flow through into the catalytic bed.

It is preferred that the oxygen containing gas additionally is added to the feed gas before the gas reaches the catalytic bed.

It is also preferred that the oxygen containing gas added to the feed gas is fed into an inert bed upstream of the catalytic bed.

Another object of the present invention is to provide a reactor for the above-mentioned method.

This object is met by a reactor for catalytic autothermal dehydrogenation of a hydrocarbon containing feed stream, preferably $C_2$-$C_4$ hydrocarbons, where hydrogen is substantially selectively oxygenated by oxygen fed in a separate oxygen containing stream, the reactor comprising a catalytic bed and optionally one or more inert bed(s) through which the carbon containing stream may flow, the reactor also comprising one or more oxygen supply tube(s) inserted in the catalytic bed, though which tubes an oxygen containing gas may be introduced directly into the catalytic bed, wherein the oxygen supply tube(s) comprises nozzles, holes or a membrane tube having pores and the nozzles, holes or pores is distributed around the circumference and the longitudinal direction of the tube.

The oxygen containing gas might be fed at any desirable location in the dehydrogenation catalytic bed, i.e. the gas might be fed at the inlet side of the catalytic bed and/or in the upper, middle and/or lower part thereof, through one or more oxygen supply tube(s) that are connected in parallel and/or in sequence.

The oxygen supply tubes might be straight and/or bent having any direction relative to the direction of flow of

3

the hydrocarbon containing gas. The oxygen containing gas may be fed through the walls and/or the ends of the oxygen supply tubes.

The dehydrogenation catalyst might be in a fixed bee, in the form of a monolith, a fluidised bed, a mixed fixed bed / fluidised bed or any combination thereof known in the art.

By feeding oxygen directly into the dehydrogenation catalytic bed the temperature gradient in the catalytic bed is reduced compared with conventional ADH reactors. Moreover, this makes it possible to reduce the costs connected to the building of the reactors.

These results might be achieved with minor effect on the product distribution compared with conventional dehydrogenation.

The enthalpy values for the dehydrogenation and oxidation reactions in question are indicated in the following table:

| Reaction | dH (900K) (kcal/mol reactant) | dH (900K) (kcal/mol $O_2$) |
|---|---|---|
| $C_3H_8 \rightarrow C_3H_6 + H_2$ | 30.9 | - |
| $2H_2 + O_2 \rightarrow 2 H_2O$ | -118.2 | -118.2 |
| $C_3H_6 + 4.5 O_2 \rightarrow 3 CO_2 + 3 H_2O$ | -460.4 | -102.3 |
| $C_3H_6 + 4 O_2 \rightarrow CO + 2 CO_2 + 3 H_2O$ | -392.8 | -98.2 |

It might be seen from these enthalpy values that the energy gain is higher from oxidation of hydrogen than of propene by using the same amount of oxygen. These results favour the selective oxidation of hydrogen in the ADH process.

It is important to distinguish between the present process from other know processes where oxygen is fed to non-converted alkane containing feed (so-called oxidative dehydrogenation). Oxidative dehydrogenation that might be described by the equation (ii) below, is a strongly exothermic reaction. As a result of this excess heat has to be taken away by means of heat exchange, which makes the process more complex.

$$C_nH_{2n+2} \rightarrow C_2H_{2n} + H_2O \qquad \text{(ii)}$$

The high ($O_2$:$C_2H_{2n+2}$) ratio as well as the absence of hydrogen leads to a lower alkene selectivity than for the present invention, de the above-mentioned DE 197 34 541.

The invention will now be illustrated through the following Examples with reference to the enclosed figures, wherein:

Figure 1 is a longitudinal section of a reactor according to the invention;
Figure 2 is a side view of a oxygen supply tube;
Figure 3 is a side view of an alternative oxygen supply tube;
Figure 4 is side view of another alternative oxygen supply tube;
Figure 5 is a graphical representation of the conversion(%) of propane as a function of time since regeneration of he catalyst;
Figure 6 is a graphical representation of the selectivety to propen as a function of time since regeneration of the catalyst; and
Figure 7 is a graphical representation of selectivity to ethene, ethane, methane, $CO_2$ and CO as a function of time since the regeneration of the catalyst.

Several tests of the present method and reactor were performed. The tests were performed at 1.05 bara and 560-625°C. The hydrocarbon feed consisted of hydrogen, propane and steam in the ratio: $H_2$ : $C_3H_8$ : $H_2O$ = 4.5 : 32 : 63.5 (mol%), with a total feed flow of 3 Nml/min (normal millilitre per minute) that was fed from the top of the reactor. The tests with the ethane dehydrogenation were performed with different ratios of ethane:$H_2$:$H_2O$ at a temperature of 600 - 675 °C. All tests was performed at a pressure of 1.05 bara. The oxygen-containing gas consisted of oxygen and nitrogen and/or steam, in various ratios. All feed components were fed through separate feed lines. Before reaching the reactor, the feed streams were preheated to 350°C. Feed and effluent gas analysis was performed by using an on-line Micro GC (Chrompack CP2002).

The dehydrogenation catalyst was a 0.25% Pt/ 0.5% Sn/Mg(Al)O catalyst described in Patent Application no. NO1998-6116 (to Statoil), which had been pelleted, crushed and sieved to a diameter of 1-1.5 mm. 30 g catalyst was used in each test. Before testing, the catalyst was *in situ* pre-treated, according to the ROR (reduction-oxidation-reduction) procedure described in NO 179 131 B (to Statoil). After approx. 1000 minutes on stream, the catalyst was regenerated according to the OR procedure described in Patent Application no. NO 1998 6116 (to Statoil).

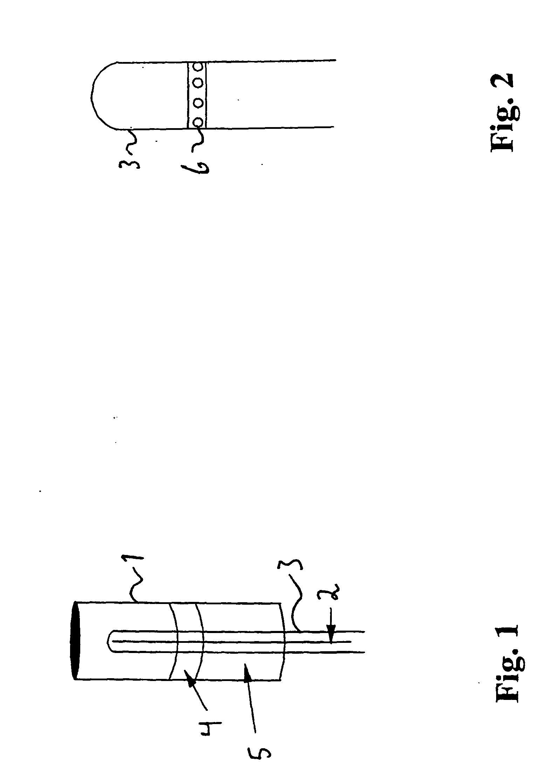

The fixed bed dehydrogenation tests were performed in a 314 steel reactor 1 as schematically illustrated in figure 1. The steel reactor 1 used in the tests had a length of 21 mm and a length of 770 mm. Four individual heating tapes (not illustrated) surrounding the reactor heated the reactor. The reactor temperature was monitored by a multi-thermocouple 2 having six measurement points, each situated 100 mm from the neighbouring measurement point. The thermocouple 2 was placed inside the oxygen feed tube 3, entering from the bottom of the reactor 1. The reactor temperature was regulated according to the highest temperature measured by one of the six thermocouples 2. The uppermost thermocouple measurement point was placed 130 mm below the reactor 1 top, while the catalyst bed 4 started 260 mm below the reactor top. The height of the catalyst bed 4 was approx. 240 mm. The volume below the catalyst bed 4 was filled with silica grains, while the volume above the catalyst bed was empty.

The oxygen-containing gas was fed through the walls of an axially inserted tube 3 (Figure 1). Three different tube configurations of the oxygen supply tube 3 were used, as illustrated in figures 2, 3 and 4.

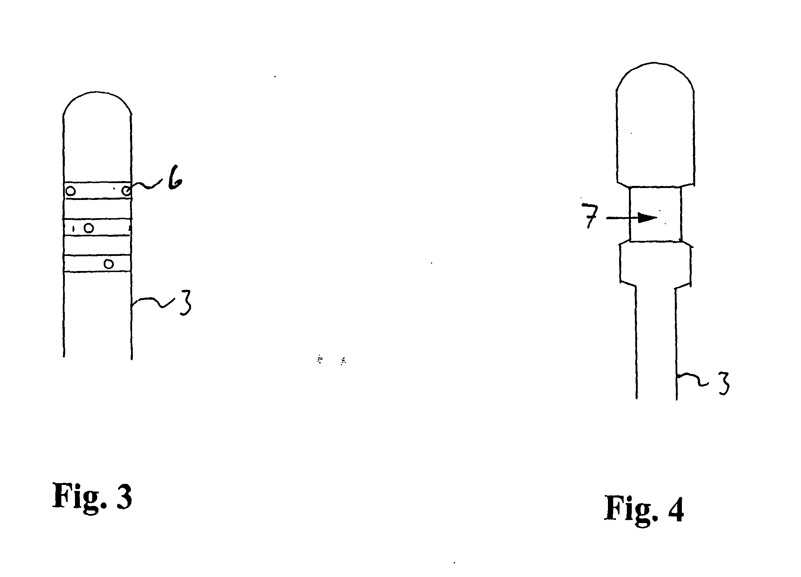

In the oxygen supply tube 3 as illustrated in figures 2 and 3, a plurality of nozzles 6 are distributed around the circumference of the tube. In the tests oxygen supply tubes 3 having tubes having nine nozzles either distributed in one height, or three nozzles in three different heights. When nozzles in three heights were used, the nozzles in one height were rotated 30° around the quartz tube compared to the nozzles in the neighbouring height. This was done to achieve the most even distribution of the oxygen containing gas in the catalytic bed.

The diameter of the nozzles 6 was 0.5 mm. Quartz sinter rings (16-40 micron pores) were welded onto the inner surface of the quartz tubes, in order to reduce the open surface area of each nozzle. In the ethane dehydrogenation tests the experiments were performed without the quartz sinter rings.

In the oxygen supply tube 3 illustrated in figure 4, a section of the oxygen supply tube 3 is substituted with a membrane tube 7 made of alumina having pore openings of 100Å.

The composition of the feed gas in the propane dehydrogenation tests was as indicated in table 1:

| Feed composition | Air (Nml/min) | $N_2$ (Nml/min) | $O_2$ in air (Nml/min) |
|---|---|---|---|
| A | 300 | 200 | 60 |
| B | 300 | 0 | 60 |
| C | 500 | 0 | 100 |
| D | 500 | 500 | 100 |
| E | 0 | 80 | 0 |
| F | 0 | 500 | 0 |
| G | 150 | 100 | 30 |
| H | 250 | 0 | 50 |
| I | 300 | 700 | 60 |
| J | 400 | 600 | 80 |

Several experiments were carried out for each type of oxygen feed tube 3. A typical test run involved testing under conventional propane dehydrogenation (PDH) conditions, followed by ADH testing with various amounts of inert gas, steam and oxygen, and finally a new test period under PDH conditions. Under PDH test conditions, a small amount of $N_2$ (80 Nml/min) was fed through the oxygen feed tube in order to avoid blocking of the nozzles or pores.

The following definitions are used for the test results in the examples below:

Propane conversion (%) = 100*sum(amount $C_x$*X)out/(sum(amount $C_x$*X)out *3* $C_3H_8$),
Propene selectivity (%)= 100*3*$C_3H_6$ut / sum(amount $C_x$*X)out, wherein $C_x$ is $C_1$ (methane. CO, $CO_2$), $C_2$ (ethane, ethene), $C_3$ (propene). The remaining selectivities are defined corresponding to-the propene selectivity.

If not otherwise indicated the oxygen supply tube illustrated in figure 2 has been used in the examples.

**Example 1. Propane dehydrogenation**

Propane dehydrogenation was carried out at 560, 600 and 625°C. $N_2$ (80 Nml/min) was fed through the oxygen supply tube 3 during the test. Typical conversion and selectivity results, obtained during a test using the nozzle tube according to figure 2, i.e. a tube having all nozzles in one height, are shown in Table 2. Similar results were obtained using the oxygen supply tubes according to figure 3 and 4, i.e. a tube having nozzles in several heights and a tube where a section is substituted by a membrane tube 7 with pores.

Table 2.

| PDH test results | | | | | |
|---|---|---|---|---|---|
| Temperature ($^{\circ}$C) | Initial or final PDH test period | Propane conversion (%) | Propene selectivity (% on $C_1$ basis) | $CO_x$ selectivity (% on $C_1$ basis) | $C_1$, $C_2$ selectivity (% on $C_1$ basis) |
| 560 | Initial | 22.5 | 99 | 0.2 | 0.8 |
| 560 | Final | 21.5 | 99 | 0.2 | 0.8 |
| Regeneration | | | | | |
| 600 | Initial | 41.5 | 97.5 | 0.7 | 1.8 |
| 600 | Final | 30.5 | 98 | 0.4 | 1.6 |
| Regeneration | | | | | |
| 600* | Initial | 35 | 98 | 0.4 | 1.6 |
| 625* | Initial | 42 | 96.5 | 1.2 | 2.3 |

*) with $N_2$ dilution (500 Nml/min fed through the nozzle tube compared to 80 Nml/min in the other tests)

It is observed that the propene selectivity is very high at all temperatures, but decreases with increasing temperatures, especially from 600 to 625$^{\circ}$C. A selectivity decrease with increasing conversion is observed. However, even at similar conversion levels, the propene selectivity is lower at 625$^{\circ}$C than at 600$^{\circ}$C. The major by-products are methane, ethane and ethene, which are formed by cracking and hydrogenation reactions. Reforming with steam in the feed generates CO and, especially, $CO_2$. Small amounts of coke formation were not measured on a regular basis, but were confirmed to be low in separate experiments.

**Example 2. Addition of oxygen-containing gas in one height**

Each test was started with fresh regenerated catalyst. The inlet of the oxygen-containing gas. i.e. the nozzles of the oxygen supply tubes, was placed 420 mm below the reactor inlet, i.e. 50 mm below the third temperature measurement point and 160 mm below the top of the catalyst bed. Typical conversion-selectivity data obtained under different conditions are shown in Table 3. All data in Table 3 were obtained approximately one hour after switching the test conditions.

Table 3.

| Conversion-selectivity data (on $C_1$ basis) obtained with a nozzle tube where the oxygen-containing gas was fed in one height. | | | | | |
|---|---|---|---|---|---|
| Oxygen feed composition (See Table 1) | Temperature (°C) | Propane conversion (%) | Propene selectivity (%) | $CO_x$ selectivity (%) | $C_1$-$C_2$ selectivity (%) |
| E (0+80) | 560 | 22.5 | 99 | 0.2 | 0.8 |
| A (300+200) | 560 | 24 | 96.3 | 2.6 | 1.1 |
| B (300+0) | 560 | 23.5 | 96.3 | 2.6 | 1.1 |
| C (500+0) | 560 | 24.5 | 93.2 | 5.1 | 1.7 |
| D (500+500) | 560 | 24.5 | 94 | 4.9 | 1.1 |
| E (0+80) | 560 | 21.5 | 99 | 0.2 | 0.8 |
| Regeneration | | | | | |
| E (0+80) | 600 | 41.5 | 97.5 | 0.7 | 1.8 |
| A (300+200) | 600 | 41 | 95.5 | 2.2 | 2.3 |
| C (500+0) | 600 | 41 | 93.5 | 3.7 | 2.8 |
| B (300+0) | 600 | 37 | 96.1 | 1.9 | 2.0 |
| E (0+80) | 600 | 35 | 98 | 0.5 | 1.5 |
| F (0+500) | 600 | 35 | 98 | 0.4 | 1.6 |
| F (0+500) | 625 | 42 | 96.5 | 1.2 | 2.3 |
| E (0+80) | 600 | 30.5 | 98 | 0.4 | 1.6 |

The initial propane conversion corresponding to an increasing oxygen amount was always higher than the final conversion of the previous set of conditions. However, in Table 3 this fact is often masked by the deactivation during the previous set of conditions. As an example, going from conditions A to C at 600°C led to a conversion increase from 39% (final measurement condition A) to 41% (first measurement condition C).

Accordingly, a decreasing oxygen amount led to a decreasing propane conversion compared to the final conversion in the previous set of conditions. As an example, going from conditions C to B at 600°C led to a conversion decrease from 38.5% (final measurement condition C) to 37% (first measurement condition B).

At all temperatures, it is observed that an increasing oxygen addition leads to a small decrease in propene selectivity and an increase in $CO_x$ selectivity. These changes are often accompanied by a smaller increase in the $C_1$-$C_2$ selectivity.

Further, it is observed that an increasing dilution of the oxygen-containing gas (given a constant propane conversion) leads to a decreasing $C_1$-$C_2$ selectivity, most probably due to a lower over-temperature. The $CO_x$ selectivity seems to be unaffected by the dilution of the oxygen-containing gas, indicating that the gas mixture is not significantly improved due to the dilution.

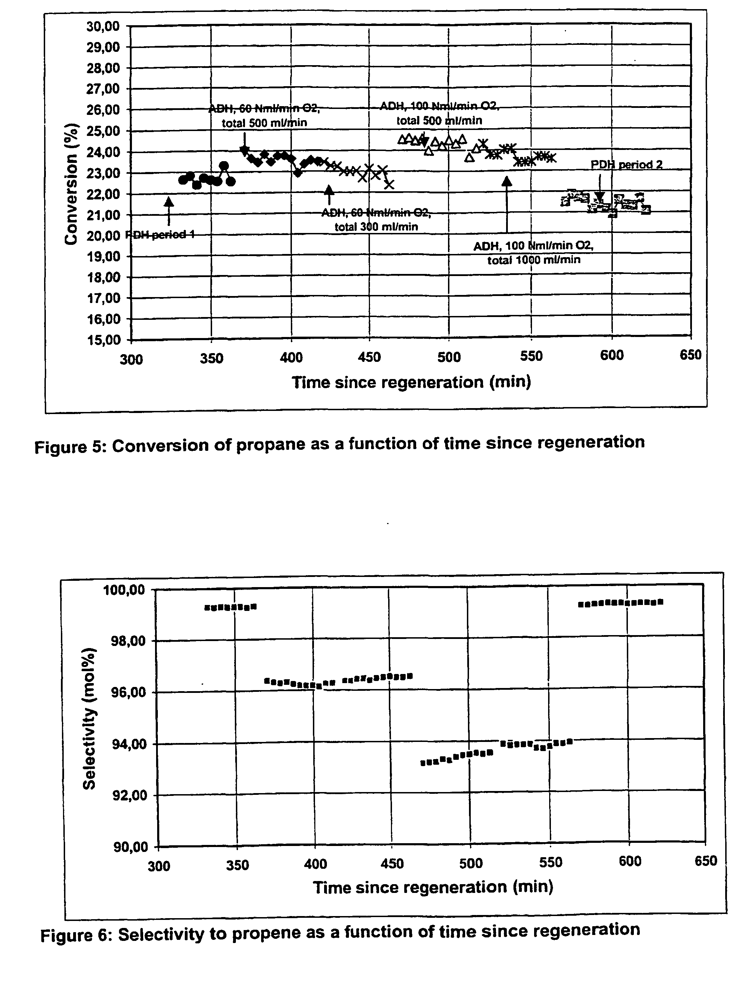

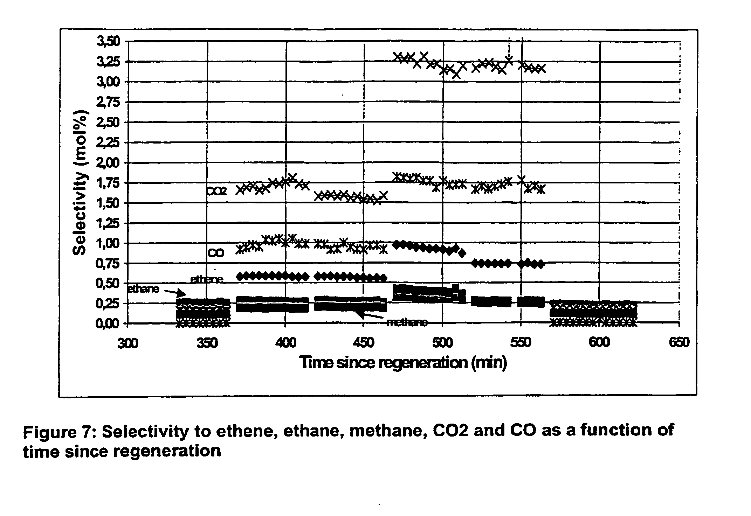

Conversion of propane and selectivity to propene in addition to the selectivity to ethene, ethane, methane, $CO_2$ and CO in these tests are illustrated as a function of time since regeneration of the in the figures 5, 6 and 7, respectively.

**Example 3. Addition of oxygen-containing gas in three heights**

The tests were carried out at 560-625°C, starting with a freshly regenerated catalyst. The first nozzle layer was placed 380mm below the reactor inlet, i.e. 10 mm below the third temperature measurement point and 120 mm below the top of the catalyst bed. Typical conversion-selectivity data obtained under different conditions are shown in Table 4. All data in Table 4 were obtained approximately 1 hour after switching the test conditions.

Table 4.

| Conversion-selectivity data (on $C_1$ basis) obtained with a nozzle tube where the oxygen-containing gas was fed in three heights. | | | | | |
|---|---|---|---|---|---|
| Oxygen feed composition (See Table 1) | Temperature (°C) | Propane conversion (%) | Propene selectivity (%) | $CO_x$ selectivity (%) | $C_1$-$C_2$ selectivity (%) |
| E (0+80) | 560 | 34 | 98.8 | 0.3 | 0.9 |
| A (300+200) | 560 | 35.5 | 97.2 | 1.8 | 1 |
| B (300+0) | 560 | 33 | 97.2 | 1.8 | 1 |
| C (500+0) | 560 | 36 | 95.2 | 3.5 | 1.3 |
| D (500+500) | 560 | 33.5 | 95.2 | 3.7 | 1.1 |
| Regeneration | | | | | |
| E (0+80) | 600 | 49 | 97 | 0.9 | 2.1 |
| A (300+200) | 600 | 38 | 96.2 | 2.2 | 1.6 |
| C (500+0) | 600 | 36.5 | 94 | 4 | 2 |
| B (300+0) | 600 | 34 | 96.5 | 2.1 | 1.4 |
| E (0+80) | 600 | 32 | 97.4 | 1.1 | 1.5 |
| F (0+500) | 600 | 30.5 | 98 | 0.9 | 1.1 |
| F (0+500) | 625 | 37 | 96.6 | 1.1 | 2.3 |
| E (0+80) | 600 | 23 | 98.1 | 0.7 | 1.2 |

As observed during Example 2 (Table 3), the initial propane conversion corresponding to an increasing oxygen amount was always higher than the final conversion of the previous set of conditions. As an example, going from conditions A to C at 600°C led to a conversion increase from 35.5% (final measurement condition A) to 36.5% (first measurement condition C). Accordingly, a decreasing oxygen amount led to a decreasing propane conversion compared to the final conversion in the previous set of conditions. As an example, going from conditions C to B at 600°C led to a conversion decrease from 36% (final measurement condition C) to 34% (first measurement condition B).

Again, it is observed that an increasing oxygen amount leads to an increase in $CO_x$ selectivity, leading to a decrease in propene selectivity. The dilution of the oxygen-containing gas has little influence on the conversion and selectivity data.

**Example 4 (theoretical example) - Steam addition**

In a separate experiment, $N_2$ in the oxygen-containing gas was partly replaced by steam. The results obtained were similar to those shown in Table 4, although a slight improvement in propene selectivity was observed, possibly due to the higher heat capacity of steam compared to nitrogen.

**Example 5. Addition of oxygen-containing gas using a porous membrane tube**

The reaction was carried out at 560-625°C, starting with a freshly regenerated catalyst. The membrane started 360 mm below the reactor inlet, i.e. 10 mm above the third temperature measurement point and 100 mm below the top of the catalyst bed. Typical conversion-selectivity data obtained under different conditions are shown in Table 5. All data in Table 5 were obtained approximately 1 hour after switching the test conditions.

**EP 1 261 570 B1**

Table 5.

| Conversion-selectivity data (on $C_1$ basis) obtained with a porous membrane tube for feeding the oxygen-containing gas. | | | | | |
|---|---|---|---|---|---|
| Oxygen feed composition (See Table 1) | Temperature (°C) | Propane conversion (%) | Propene selectivity (%) | $CO_x$ selectivity (%) | $C_1$-$C_2$ selectivity (%) |
| E (0+80) | 560 | 38 | 98 | 1 | 1 |
| A (300+200) | .560 | 43.5 | 95.5 | 3.6 | 0.9 |
| I (300+700) | 560 | 44 | 96 | 3.1 | 0.9 |
| J (400+600) | 560 | 47 | 96 | 3.1 | 0.9 |
| D (500+500) | 560 | 46.5 | 95.7 | 3.2 | 1.1 |
| E (0+80) | 560 | 38.5 | 96.9 | 2.2 | 0.9 |
| Regeneration | | | | | |
| E (0+80) | 600 | 51 | 96.1 | 2.1 | 1.7 |
| A (300+200) | 600 | 48 | 94.6 | 3.7 | 1.7 |
| I (300+700) | 600 | 42.5 | 95.5 | 3.3 | 1.2 |
| J (400+600) | 600 | 49 | 95 | 3.2 | 1.8 |
| E (0+80) | 600 | 38 | 95.5 | 3.2 | 1.3 |

During the tests with a membrane tube, a steep temperature increase was observed (using the thermocouples that were placed inside the membrane) upon oxygen feeding: After only 1 minute with oxygen feed A at 600°C, the temperature measured inside the tube was 700°C. Such a temperature increase was not observed during the nozzle tube tests. Combined with the observation that the $C_1$-$C_2$ selectivity was as expected for the original temperature (Table 5 compared to Tables 3 and 4), this result may suggest that a significant part of the oxidation reaction took place inside the membrane tube. Better temperature control was obtained with an increased dilution of the oxygen-containing gas. This result may suggest that a net flow of oxygen-containing gas into the outer tube is obtained with an increased total gas flow.

As observed in the previous Examples, oxygen addition leads to an increasing propane conversion and decreasing propene selectivity. However, the effect is less pronounced than for the nozzle tubes. The propene selectivity observed under ADH conditions is higher at 560°C than at 600°C for similar propane conversions.

**Example 6 - Dehydrogenation in a fluidised bed reactor**

One PDH-ADH experiment was carried out in a fluid bed reactor. The reactor was made of quartz, with inner diameter 23 mm. The reactor was heated by a tubular furnace.

The feed composition as well as the catalyst were the same as used in the fixed bed tests; however, the catalyst was ground to a particle size of 45-90 microns. The hydrocarbon feed mixture was fed through a sinter plate at the bottom of the reactor. The total gas flow was 300 Nml/min.

The catalyst (190g) was diluted with sintered alumina (Condea Puralox, calcined at 1350 °C/12 hours, 12 ml, 20,54 g, 45-90 mikron). The oxygen-containing gas was fed through a quartz tube with 9 nozzles in one height. The nozzle construction was as shown in Figure 2a). The nozzles were placed 30 mm above the start of the catalyst bed. The total bed height (stagnant catalyst) was 60 mm. During PDH conditions 50 ml/min $N_2$ was fed through the oxygen supply tube. The test was carried out in the bubble flow regime. The temperature profile in the reactor was measured by using a thermocouple inside the oxygen supply tube. The measure point was 5 mm above the oxygen feed nozzles. A quartz sinter was placed above the catalyst bed, before the end of the isothermal zone, in order to prevent catalyst fines from entering the colder catalyst disengagement zone and thereby influencing the propene yield. The exit gas composition was measured by using an on-line Mikro-Gc (HP QUADH). The tests were started using fresh regenerated catalyst. Each set of conditions was studied for one hour without any intervening regeneration. The results are shown in table 6.

Table 6.

| Conversion and selectivity data (on $C_1$ basis) for PDH - ADH tests in a fluidised bed reactor. The oxygen containing gas was fed through nine nozzles at the same height. | | | | | | |
|---|---|---|---|---|---|---|
| Temperature (°C) | Oxygen feed composition (Nml/min) | | Propane conversion (%) | Propene selectivity (%) | $CO_x$ selectivity (%) | $C_1$, $C_2$ selectivity (%) |
| | Air | $N_2$ | | | | |
| 600 | 0 | 50 | 25,3 | 93,0 | 1,4 | 5,4 |
| 600 | 50 | 50 | 25,3 | 87,4 | 4,8 | 5,3 |
| 600 | 30 | 70 | 12,8 | 86,6 | 4,8 | 7,7 |
| 600 | 0 | 50 | 12,0 | 88,4 | 2,3 | 11,7 |

The results in table 6 illustrates that the $CO_x$ selectivity increased by 3,4% compared with PDH conditions when 50 ml/min air was fed into the reactor. 30 ml air resulted in an increase in $CO_x$ selectivity of 2,5% . This is close to the results obtained in the fixed bed reactor (table 3). It is expected that an even better results could be obtained in a larger fluid bed reactor, where gas phase oxidation could be limited by going from a bubbling to a turbulent flow regime.

**Example 7 - Ethane dehydrogenation (EDH)**

Test on ethane dehydrogenation was performed i a fixed bed reactor as described above at 600 - 725 °C and 1,05 bara. The same amount of catalyst and particle size as described under the tests for dehydrogenation of propane in a fixed bed reactor was used. Some test results are shown in table 7. The results illustrates that the catalyst has high selectivity for ethene.

Table 7.

| Data for conversion and selectivity for ethane dehydrogenation at 600-725°C. | | | | | | | |
|---|---|---|---|---|---|---|---|
| Temp. (°C) | Ethane feed (ml/min) | $H_2$ feed (ml/min) | $H_2O$-feed (ml/min) | Ethane conv. (%) | Ethene select. (%) | $CO_x$ select. (%) | $C_1$,$C_3$ select. (%) |
| 600 | 450 | 300 | 600 | 7 | 77 | 11 | 12 |
| 650 | 600 | 180 | 600 | 25 | 91 | 4 | 5 |
| 700 | 1200 | 360 | 600 | 35 | 89 | 5 | 6 |
| 725 | 1800 | 360 | 600 | 34 | 89 | 5 | 6 |

**Example 8 - Autothermal ethane dehydrogenation (AEDH)**

Autothermal ethane dehydrogenation tests were performed in the same reactor and under the same conditions as in Example 7. The oxygen containing gas was fed from a quarts tube having nine nozzles in three heights, as described above and illustrated in figure 3. However, the oxygen supply tube had no sintered quarts rings, which means that the opening of the nozzles was as defined by the holes in the quarts tube, i.e. 0.5 mm in diameter.
Some test results are shown in table 8. The results in table 8 demonstrates that an increase in the oxygen content in the feed gas resulted in an increased conversion and at the same time a higher $CO_x$ selectivity. This tendency is confirmed by a comparison with the results in table 7. It should be noted that the catalyst was deactivated under EDH and EADH conditions too, so that the change in the conversion under the different conditions in not absolute.

Table 8.

| Temp. (°C) | Etan feed (ml/ min) | $H_2$ feed (ml/min) | $H_2O$ feed (ml/min) | Luft feed (ml/min) | Etan conv (%) | Ethene select. (%) | $CO_x$ select. (%) | $C_1,C_3$ select. (%) |
|---|---|---|---|---|---|---|---|---|
| 650 | 600 | 180 | 600 | 375 | 30 | 70 | 20 | 10 |
| 650 | 600 | 180 | 600 | 190 | 20 | 77 | 14 | 9 |
| 700 | 1200 | 360 | 600 | 500 | 57 | 65 | 20 | 15 |
| 700 | 1200 | 360 | 600 | 375 | 43 | 85 | 8 | 7 |

Data for conversion and selectivity obtained under autothermal ethane dehydrogenation at 650 - 700 °C.

**Example 9 (theoretical example) - Addition of oxidation selectivity modifiers**

The potential of some materials as oxidation selectivity tuning catalysts was investigated in the reactor containing an oxygen supply tube with nine nozzles in three heights, and with quartz sinter rings welded to its inner surface. The catalyst used in Examples 1-8 was modified by either 2 wt% Ni, 6 wt% Pt, or 3 wt% Mo. Each catalyst led to a significant reduction of the propene selectivity during ADH operation compared to the results shown in Table 4, and thus clearly indicated the potential of these additives as oxidation selectivity modifiers.

**Discussion**

In previous ADH process studies, the heat distribution has been restricted by the assumed need to mix the alkane-containing gas and the oxygen-containing gas outside the dehydrogenation catalyst bed. The oxidation reaction will then take place in a narrow band of the reactor, and the subsequent dehydrogenation reaction will result in falling temperature in the reaction mixture as it flows through the catalytic bed.

At 600°C and with the feed composition used in this work, the equilibrium propane conversion is close to 60%. The amounts of oxygen fed to the reactor corresponds to 60% and 100%, respectively, of the oxygen amount required to obtain autothermal operation at 42% conversion, i.e.; close to the conversion numbers obtained in this work. The calculated loss in propene selectivity for 90%, 50% and 0% selectivity for hydrogen oxidation, respectively, has been calculated and is shown in Table 9.

Table 9.

| Oxygen amount (Nml/min) | $H_2$ oxidation selectivity (%) | Propane conversion (% on $C_1$ basis) | Propene selectivity loss (% on $C_1$ basis) |
|---|---|---|---|
| 60 | 0 | 20 | 7.8 |
| 60 | 0 | 40 | 3.9 |
| 100 | 0 | 20 | 13.0 |
| 100 | 0 | 40 | 6.5 |
| 60 | 50 | 20 | 3.9 |
| 60 | 50 | 40 | 2.0 |
| 100 | 50 | 20 | 6.5 |
| 100 | 50 | 40 | 3.3 |
| 60 | 90 | 20 | 0.8 |
| 60 | 90 | 40 | 0.4 |
| 100 | 90 | 20 | 1.3 |
| 100 | 90 | 40 | 0.7 |

Calculated loss in propene selectivity upon oxygen addition, assuming that propene, not propane, is oxidised.

In the calculations, it was assumed that the initial (PDH) propene selectivity is 100%, and that the oxidation of propene leads to a CO/CO$_2$ ratio of ½, in agreement with the experimental results. It was further assumed that propene, not propane, is oxidised.

A comparison between Table 9 and the experimental results indicate that the selectivity for hydrogen oxidation is between 50 and 90% in all tests performed in this work. The smallest selectivity loss was obtained with the membrane tube, see fig. 4, at 600°C, with significant dilution of the oxygen-containing gas. With less dilution of the oxygen-containing gas, a significant selectivity loss was observed, see Table 5. The corresponding temperature profiles suggested that with less dilution, most of the oxidation reactions take place within the membrane tube, i.e.; outside the catalyst bed. This result clearly indicates that the dehydrogenation catalyst used in this work is active as a selective hydrogen oxidation catalyst.

The lowest alkene selectivity loss obtained during ADH in the membrane reactor, Example 4, corresponds to a hydrogen oxidation selectivity close to 90%, i.e. slightly better than the best result obtained in the applicant's previous patent application WO96/19424, where the oxygen-containing gas was mixed with the hydrocarbon-containing gas before entering the (second) dehydrogenation chamber. It was here assumed that premixing of the hydrocarbon- and oxygen-containing gases before the catalyst bed was necessary in order to obtain good mixing of the gases, and thus to avoid a shift in oxidation selectivity due to local over-concentrations of hydrocarbons after partially oxidising local hydrogen.

The results obtained in the present application indicate that sufficient mixing may be obtained inside the dehydrogenation catalyst bed, as long as the gas velocity out of the oxygen-containing feed tube is sufficiently large. Sufficient gas velocities were obtained by diluting the oxygen-containing gas. A similar effect could have been obtained by using either smaller nozzles, a membrane with a smaller pore size, or a larger total gas feed. The slightly higher hydrogen oxidation selectivity obtained in this work compared to WO96/19424 is probably due to a minimisation of unselective gas phase reactions between oxygen and the hydrogen-hydrocarbon gas mixture. One may speculate that the addition of a dehydrogenation catalyst to the surface of the oxygen feed tube, thus minimising the gas phase contact time even further, would lead to a further increase in alkene selectivity.

The temperature effect on product selectivity is indicated by the results in Example 3, where similar conversions are obtained at 560 and 600°C. The selectivity loss due to oxygen addition is similar at the two temperatures. However, due to the higher propene selectivity obtained under PDH conditions at 560°C compared to 600°C, the overall propene selectivity is higher at 560 than at 600°C, even under ADH conditions. This result suggests that a low reaction temperature, as well as limited hot spots in the catalyst bed, is advantageous for the overall alkene selectivity.

The catalyst deactivation observed under ADH conditions is either similar, or smaller, compared to PDH conditions. This effect is probably due to less carbon deposition with an increasing (H+O)/C ratio in the gas mixture, and represents an additional advantage of the ADH process.

Typical characteristics for the tests of the above Examples are:

(i) a high selectivity for propene was observed under all conditions (PDH, EDH and ADH using different amount of oxygen and inert gas)
(ii) a high selectivity for combustion of hydrogen was observed under ADH conditions
(iii) some deactivation of the catalyst was observed during the test runs
(iv) an increasing amount of oxygen fed to the reactor led a simultaneous increase in the conversion of propane and a slight reduction in propene selectivity
(v) CO$_2$ was the major by-product under ADH conditions. However, an increase in CO and ethene selectivity compared to PDH test conditions was also observed

The results obtained in this work lead to the following general conclusions:
Addition of an oxygen-containing gas directly into a catalyst bed active for alkane dehydrogenation leads to an increasing alkane conversion. Simultaneously, limited alkene selectivity drop is observed. The alkene yield increases with an increasing oxygen feed distribution, by maintaining a sufficient velocity of the oxygen-containing gas, and by adding the oxygen-containing gas after sufficient conversion of the alkane. The addition of an oxygen-containing gas further leads to a decrease in catalyst deactivation. Finally, it has been observed that the catalyst used in the present study is an excellent catalyst for selective hydrogen oxidation. The oxidation selectivity may be tuned by adding appropriate catalyst modifiers.

Autothermal dehydrogenation, performed by adding the oxygen-containing gas directly into the dehydrogenation catalyst bed, represents a much simpler reactor design compared to competing designs. It also leads to high alkene yields, high catalyst stability and improved energy efficiency compared to conventional alkane dehydrogenation processes.

A relative specific catalyst, previous known from NO 1998 6116, has been used in the above examples. However, it is clear that other catalysts fulfilling the requirements for selectivity both with regard to alkane dehydrogenation and hydrogen oxidation may be used. The materials most used in catalysts are noble metals such as platinum, or chromium on a catalyst support. Materials such as alumina, zinc aluminate and calcinated hydrocalcite might be used as catalyst support.

One or more promoters, such as Sn, Th, Ga, alkaline metal oxides or earth alkaline metal oxides are often added to the catalyst.

It is important that the catalyst selectively supports selective oxidation of hydrogen in the presence of hydrocarbons, to avoid the competing combustion of hydrocarbons. Platinum and tin on calcinated hydrocalsite, platinum and tin on alumina and Pt/Sn/Mg(Al)O are examples on preferred catalysts.

Mixed catalytic beds comprising both a selective alkane hydrogenation catalyst and a selective hydrogen oxidation catalyst may also be used.

It is also applicable to mix a part of the total amount of oxygen containing gas into the feed gas before it enters the catalytic bed. Preferably, any oxygen containing gas added to the feed gas before it enters the catalytic bed is preferably fed into a bed of inert particles, such as beads, pellets and the like. A part of the hydrogen and possibly a part of the hydrocarbon present in the feed gas will then be burned before it enters the catalytic bed so that the feed gas is preheated. A separate heating unit for the feed gas may then be unnecessary.

Steam has been added to the feed gas before entering the reactor in all examples. The purpose of the steam is to reduce coke formation at the catalyst. The use of steam is preferred but not essential as it may be omitted in systems where formation of coke is tolerated or where the catalyst has to be fired relatively often, or if substantial amounts of hydrogen are mixed with the feed gas.

The invention has been described with reference to laboratory reactors and test. In full-scale plants the reactor will normally include a number of oxygen supply tubes. Full-scale plants may also comprise a plurality of parallel or serially connected reactors.

**Claims**

1. A method for autothermal or substantially autothermal catalytic dehydrogenation of hydrocarbons, where a hydrocarbon containing feed gas optionally is mixed with steam and optionally mixed with hydrogen, is preheated and led into a reactor comprising a dehydrogenation catalytic bed and where a oxygen containing gas is fed into the hydrocarbon containing feed gas directly in the catalytic bed, **characterised** i n that the oxygen containing gas is fed into the catalytic bed from one or more oxygen supply tube(s) (3) having a plurality of openings distributed in the catalytic bed.

2. Method according to claim 1, **characterised in that** the openings in a oxygen supply tube are situated around the circumference and in the longitudinal direction of the oxygen supply tube (3).

3. Method according to claim 1, **characterised in that** the openings in the oxygen supply tube is a membrane having pores allowing oxygen to flow through into the catalytic bed.

4. Method according any of the preceding claims, **characterised in that** the oxygen containing gas additionally is added to the feed gas before the gas reaches the catalytic bed.

5. Method according to claim 4, **characterised in that** the oxygen containing gas added to the feed gas is fed into an inert bed upstream of the catalytic bed.

6. Reactor for catalytic autothermal dehydrogenation of a hydrocarbon containing feed stream, preferably $C_2$-$C_4$ hydrocarbons, where hydrogen is substantially selectively oxygenated by oxygen fed in a separate oxygen containing stream, the reactor comprising a catalytic bed (4) and optionally one or more inert bed(s) through which the carbon containing stream may flow, the reactor also comprising one or more oxygen supply tube(s) (3) inserted in the catalytic bed (4), though which tubes (3) an oxygen containing gas may be introduced directly into the catalytic bed (4),
**characterised in that** the oxygen supply tube(s) (3) comprises nozzles, holes (6) or a membrane tube (7) having pores and the nozzles, holes or pores is distributed around the circumference and the longitudinal direction of the tube (3).

7. Reactor according to claim 6, **characterised in that** the nozzles or holes in the oxygen supply tube (3) are evenly distributed around the circumference and in the longitudinal direction of the tube to cause as uniform sa possible distribution of the oxygen containing gas in the catalytic bed.

8. Reactor according to claim 6 or 7, **characterised in that** it comprises a plurality of oxygen supply tubes for feeding oxygen containing gas into the catalytic bed.

**Patentansprüche**

1. Verfahren zur autothermischen oder im wesentlichen autothermischen katalytischen Dehydrogenierung von Kohlenwasserstoffen, wobei ein kohlenwasserstoffhaltiges Einsatzgas wahlweise mit Dampf und wahlweise mit Wasserstoff gemischt wird, vorgewärmt wird und in einen Reaktor geleitet wird, der ein Dehydrierungskatalysatorbett aufweist, und wobei direkt in dem Katalysatorbett ein sauerstoffhaltiges Gas in das kohlenwasserstoffhaltige Einsatzgas eingespeist wird, **dadurch gekennzeichnet, dass** das sauerstoffhaltige Gas von einer oder mehreren Sauerstoffzuleitung(en) (3), die mit mehreren in dem Katalysatorbett verteilten Öffnungen versehen sind, in das Katalysatorbett eingespeist wird.

2. Verfahren nach Anspruch 1, **dadurch gekennzeichnet, dass** die Öffnungen in einer Sauerstoffzuleitung um den Umfang und in der Längsrichtung der Sauerstoffzuleitung (3) angeordnet sind.

3. Verfahren nach Anspruch 1, **dadurch gekennzeichnet, dass** die Öffnungen in der Sauerstoffzuleitung eine Membran darstellen, die Poren aufweist, die es ermöglichen, dass Sauerstoff hindurch in das Katalysatorbett strömt.

4. Verfahren nach einem der vorhergehenden Ansprüche, **dadurch gekennzeichnet, dass** das sauerstoffhaltige Gas zusätzlich zu dem Einsatzgas hinzugefügt wird, bevor das Gas das Katalysatorbett erreicht.

5. Verfahren nach Anspruch 4, **dadurch gekennzeichnet, dass** das sauerstoffhaltige Gas, das dem Einsatzgas hinzugefügt wird, in ein inertes Bett stromaufwärts des Katalysatorbettes eingespeist wird.

6. Reaktor zur katalytischen autothermalen Dehydrogenierung eines Kohlenwasserstoffe enthaltenden Einsatzstoffstroms, vorzugsweise von $C_2$-$C_4$-Kohlenwasserstoffen, wobei Wasserstoff durch Sauerstoff, der in einem separaten sauerstoffhaltigen Strom eingespeist wird, im wesentlichen selektiv oxidiert wird, wobei der Reaktor ein Katalysatorbett (4) und wahlweise ein oder mehrere inerte Betten aufweist, durch welche der kohlenstoffhaltige Strom strömen kann, wobei der Reaktor außerdem eine oder mehrere Sauerstoffzuleitung(en) (3) aufweist, die in das Katalysatorbett (4) eingesetzt sind, wobei durch diese Leitungen (3) ein sauerstoffhaltiges Gas direkt in das Katalysatorbett (4) eingebracht werden kann, **dadurch gekennzeichnet, dass** die Sauerstoffzuleitung(en) (3) Düsen, Löcher (6) oder ein Membranrohr (7) mit Poren aufweist bzw. aufweisen und die Düsen, Löcher oder Poren um den Umfang und in Längsrichtung der Leitung (3) verteilt sind.

7. Reaktor nach Anspruch 6, **dadurch gekennzeichnet, dass** die Düsen oder Löcher in der Sauerstoffzuleitung (3) um den Umfang und in der Längsrichtung der Leitung gleichmäßig verteilt sind, um eine Verteilung des sauerstoffhaltigen Gases in dem Katalysatorbett zu bewirken, die so gleichmäßig wie möglich ist.

8. Reaktor nach Anspruch 6 oder 7, **dadurch gekennzeichnet, dass** er mehrere Sauerstoffzuleitungen aufweist, um sauerstoffhaltiges Gas in das Katalysatorbett einzubringen.

**Revendications**

1. Procédé de déshydrogénation catalytique autothermique ou sensiblement autothermique d'hydrocarbures, dans lequel un gaz d'alimentation contenant un hydrocarbure est facultativement mélangé avec de la vapeur d'eau et facultativement mélangé avec de l'hydrogène, est préchauffé et amené dans un réacteur comprenant un lit catalytique de déshydrogénation et dans lequel un gaz contenant de l'oxygène est amené dans le gaz d'alimentation contenant un hydrocarbure directement dans le lit catalytique, **caractérisé en ce que** le gaz contenant de l'oxygène est introduit dans le lit catalytique à partir d'un ou de plusieurs tuyaux d'alimentation en oxygène (3) présentant une pluralité d'orifices répartis dans le lit catalytique.

2. Procédé selon la revendication 1, **caractérisé en ce que** les orifices d'un tuyau d'alimentation en oxygène se situent sur la circonférence et dans la direction longitudinale du tuyau d'alimentation en oxygène (3).

3. Procédé selon la revendication 1, **caractérisé en ce que** les orifices du tuyau d'alimentation en oxygène consti-

**EP 1 261 570 B1**

tuent une membrane présentant des pores permettant à l'oxygène de traverser le lit catalytique.

4. Procédé selon l'une quelconque des revendications précédentes, **caractérisé en ce que** le gaz contenant de l'oxygène est en outre ajouté au gaz d'alimentation avant que le gaz n'atteigne le lit catalytique.

5. Procédé selon la revendication 4, **caractérisé en ce que** le gaz contenant de l'oxygène ajouté au gaz d'alimentation est introduit dans un lit inerte en amont du lit catalytique.

6. Réacteur de déshydrogénation catalytique autothermique d'un courant d'alimentation contenant des hydrocarbures, de préférence des hydrocarbures en $C_2$-$C_4$, dans lequel de l'hydrogène est oxygéné sensiblement sélectivement par l'oxygène introduit dans un courant séparé contenant de l'oxygène, le réacteur comprenant un lit catalytique (4) et facultativement un ou plusieurs lits inertes pouvant être traversés par le courant contenant du carbone, le réacteur comprenant également un ou plusieurs tuyaux d'alimentation en oxygène (3) insérés dans le lit catalytique (4), à travers lesquels tuyaux (3) un gaz contenant de l'oxygène peut être introduit directement dans le lit catalytique (4), **caractérisé en ce que** le ou les tuyaux d'alimentation en oxygène (3) comprennent des buses, des cavités (6) ou un tuyau à membrane (7) présentant des pores et les buses, cavités ou pores étant répartis sur la circonférence et dans la direction longitudinale du tuyau (3).

7. Réacteur selon la revendication 6, **caractérisé en ce que** les buses ou cavités dans le tuyau d'alimentation en oxygène (3) sont réparties uniformément sur la circonférence et dans la direction longitudinale du tuyau afin d'obtenir une répartition aussi uniforme que possible du gaz contenant de l'oxygène dans le lit catalytique.

8. Réacteur selon la revendication 6 ou 7, **caractérisé en ce qu'**il comprend une pluralité de tuyaux d'alimentation en oxygène pour introduire le gaz contenant de l'oxygène dans le lit catalytique.

**Fig. 1**

**Fig. 2**

EP 1 261 570 B1

**Fig. 4**

**Fig. 3**

17

**Figure 5: Conversion of propane as a function of time since regeneration**

**Figure 6: Selectivity to propene as a function of time since regeneration**

**Figure 7: Selectivity to ethene, ethane, methane, CO2 and CO as a function of time since regeneration**